Europäisches Patentamt

European Patent Office

Office européen des brevets

(11) Publication number: **0 289 947**
**A1**

## EUROPEAN PATENT APPLICATION

(21) Application number: 88106893.6

(22) Date of filing: 29.04.88

(51) Int. Cl.4: **C12N 15/00 , C12N 1/20 ,**
**//(C12N1/20,C12R1:41)**

The applicant has filed a statement in accordance with Rule 28 (4) EPC (issue of a sample only to an expert). Accession number(s) of the deposit(s): ATCC 53611 and 53612.

(30) Priority: 30.04.87 US 44401

(43) Date of publication of application:
**09.11.88 Bulletin 88/45**

(84) Designated Contracting States:
**AT BE CH DE ES FR GB GR IT LI LU NL SE**

(71) Applicant: **THE GENERAL HOSPITAL CORPORATION**
**55 Fruit Street**
**Boston MA 02114(US)**

(72) Inventor: **Wilson, Kate**
**80 Lowden Avenue No. 2**
**Somerville, MA 02144(US)**

(74) Representative: **Klein, Otto, Dr. et al**
**Hoechst AG Zentrale Patentabteilung**
**Postfach 80 03 20**
**D-6230 Frankfurt am Main 80(DE)**

(54) **Nodulation and nitrogen fixing rhizobial mutants.**

(57) A Tn5-mutagenized rhizobial strain which is incapable of fixing nitrogen.

EP 0 289 947 A1

# NODULATION AND NITROGEN FIXING RHIZOBIAL MUTANTS

## BACKGROUND OF THE INVENTION

### Field of the Invention

This invention belongs to the field of bacterial genetics.

### Description of the Background Art

Specific strains of rhizobia enter into nitrogen-fixing symbioses with specific host plants, almost exclusively legumes. Successful recognition between compatible bacterial and plant partners elicits the development of a novel plant organ, the root nodule. The bacterial invade the developing nodule and there differentiate into the nitrogen-fixing "bacteroid" form (reviewed in references 21 and 73).

There are two major groups of rhizobia, commonly known as the fast-and the slow-growing species. These two groups differ by many biochemical and physiological criteria and have recently been placed in two separate genera, Rhizobium and Bradyrhizobium, respectively, (36). The two groups also differ in several symbiotic properties. For example, Bradyrhizobium strains can be induced to fix nitrogen in free-living culture (42, 47, 54), whereas Rhizobium species will only fix nitrogen symbiotically; fast-growing species generally infect only a few, closely related legumes, whereas Bradyrhizobium species of the cowpea cross-inoculation group can infect a broad range of diverse legume hosts (25); fast-growing species almost invariably infect their hosts via the root hairs, whereas a single slow-growing species may infect one host, such as pigeonpea, by the root hairs, and another, such as groundnut, by direct intercellular penetration between the epidermal cells--so-called crack entry (17, 21, 37).

Significant process has been made in the identification of bacterial genes that are required for the induction of nodules (nod genes) and the fixation of nitrogen (fix genes) in the fast-growing Rhizobium species (reviewed in references 4 and 41). In contrast, considerably less is known about the bacterial genes required for the establishment of the symbiosis by slow-growing Bradyrhizobium species. To date, the most successful approach used in the study of Bradyrhizobium symbiotic genes has been to look for genes which share structural or functional homology with previously identified Rhizobium symbiotic genes. Thus, common nodulation genes, the nitrogenase structural genes, and the homologs of other Rhizobium genes required for nitrogen fixation have been identified in Bradyrhizobium species (28, 32, 46, 51, 62, 63). However, this approach is unlikely to identify all genes that are responsible for the differences in host range or for other symbiotic differences between the two groups of rhizobia. An alternative approach to the identification of such genes is random mutagenesis followed by screening for symbiotic mutants on one or more host plants.

There are several reports of Bradyrhizobium japonicum (the endosymbiont of soybean ) mutants which were isolated by chemical or by UV mutagenesis (20, 44, 69); more recently, transposon Tn5 has been used both for random (16, 34, 58) and for localized (30, 35, 46) mutagenesis in several Bradyrhizobium species. However, the majority of these reports concern B. japonicum, and in each case mutants were only screened on a single host plant.

### Summary of the Invention

In this application is described the Tn5 mutagenesis of Bradyrhizobium sp. (Arachis) strain NC92 (a member of the cowpea cross-inoculation group), and the screening of putative symbiotic mutants on three host plants, siratro (Macroptilium atropurapureum), pigeonpea (Cajanus cajan), and groundnut (Arachis hypogaea). These particular host species were chosen because the latter two show different modes of Bradyrhizobium infection (root hair and crack entry infection, respectively), and siratro is a small-seeded legume that is routinely used for screening Bradyrhizobium strains in test tube assays.

Thus, screening methods are described which allow ready identification of desired mutants.

Two unusual types of symbiotic mutants are disclosed. In particular, a host-specific nodulation mutant, and a novel class of symbiotic mutants which have host-specific defects in nitrogen fixation are disclosed.

## DESCRIPTION OF THE PREFERRED EMBODIMENTS

## Transposon Tn5 mutagenesis of Bradyrhizobium sp. (Arachis) strain NC92.

Random Tn5 mutagenesis is carried out by using the "suicide" plasmid pGS9 (64), and kanamycin-resistant NC92 transconjugants are obtained at a frequency of approximately 1 in $10^5$ NC92 recipients.

More than 900 of these $Km^R$ NC92 transconjugants were individually screened for their symbiotic phenotypes on groundnut and on siratro. The screening on siratro is only partially successful in identifying Fix$^-$ mutants, because the restricted growth of the plants in small tubes results in all plants looking somewhat unhealthy. Nevertheless, one Fix$^-$ mutant (NC92 no. 748) and one Nod$^-$ mutant (NC92 no. 21) were identified. In contrast, groundnuts inoculated with NC92 symbiotic mutants can easily be recognized by their pale green, nitrogen-starved appearance after about 6 weeks of growth. Putative Fix$^-$ and Nod$^-$ NC92 mutants are further tested in replicated trials on three NC92 host plants, groundnut, pigeonpea, and siratro (at least two independent trials per mutant). Data from a representative trial are given in Table 1:

TABLE 1. **Phenotypes of plants inoculated with putative Bradyrhizobium sp. strain NC92 symbiotic mutants[a]**

| Plant | Strain inoculated | Leaf color | Shoot wt (g) | Total N[b] (mg) | Relative N fixed (%) | Ara |
|---|---|---|---|---|---|---|
| Groundnut | NC92 | Dark | 5.4 | 135.9 | 100 | + |
| | 284 | Dark | 3.8 | 83.0[c] | 43 | + |
| | 302 | Pale | 3.5 | 54.7[c] | 13 | + |
| | 639 | Pale | 3.6 | 46.0[c] | -- | - |
| | 727 | Pale | 3.8 | 60.3[c] | 19 | + |
| | 748 | Dark | 5.0 | 124.4 | 88 | + |
| | 807 | Pale | 3.1 | 38.4[c] | -- | - |
| | 831 | Dark | 4.5 | 102.4[c] | 64 | + |
| | 853 | Dark | 3.8 | 89.5[c] | 50 | + |
| | 868 | Dark | 4.9 | 102.3[c] | 64 | + |
| | 979 | Dark | 4.5 | 107.6[c] | 70 | + |
| Pigeonpea | NC92 | Dark | 583 | 15.9 | 100 | + |
| | 284 | Pale | 206 | 2.8[c] | 6 | + |
| | 302 | Pale | 246 | 3.7[c] | 13 | + |
| | 639 | Pale | 167 | 1.7[c] | -- | - |
| | 727 | Dark | 651 | 15.4 | 96 | + |

[a]The data are presented as the mean of four replications. Values are given per pot, i.e., per two plants. N refers to combined nitrogen. The acetylene reduction activity is defined as (+) when the average activity over all experiments was greater than 30% of the wild type and (-) when the average activity was less than 3% of the wild type. All numeral data were subjected to analysis of variance, and in each case treatment differences were found to be highly significant ($\underline{P}$ 0.01). The data for total N were subjected to a two-tailed Dunnett test for comparing a control mean to each other group mean (74). The standard errors of the mean were as follows for groundnut, pigeonpea, and sirato, respectively: ±0.3, ±66, and ±45 mg for shoot weight and ±6.1, ±1.8, and ±1.8 mg for total N.

[b]The least significant differences for total N at 1% level of significance are as follows: for groundnut, ±21.4 mg; for pigeonpea and siratro, ±6.3 mg.
[c]The treatment mean differs from the wild-type control at a level of significance of 1%.

|         | 748[d] | Pale   | 207 | 2.4[c]  | 4   | - |
|---------|--------|--------|-----|---------|-----|---|
|         | 807    | Pale   | 171 | 2.1[c]  | --  | - |
|         | 831    | Medium | 427 | 9.2[e]  | 52  | + |
|         | 853    | Medium | 457 | 9.2[e]  | 52  | + |
|         | 868    | Dark   | 446 | 9.3[e]  | 53  | + |
|         | 979    | Medium | 240 | 3.4[c]  | 11  | + |
| Siratro | NC92   | Dark   | 517 | 19.3    | 100 | + |
|         | 284    | Dark   | 327 | 11.0[c] | 55  | + |
|         | 302    | Pale   | 53  | 0.8[c]  | 1   | + |
|         | 639    | Pale   | 42  | 0.6[c]  | --  | - |
|         | 727    | Dark   | 437 | 14.4    | 74  | + |
|         | 748    | Pale   | 50  | 0.7[c]  | 1   | - |
|         | 807    | Pale   | 50  | 0.6[c]  | --  | - |
|         | 831    | Dark   | 374 | 12.1[c] | 62  | + |
|         | 853    | Dark   | 278 | 8.6[c]  | 43  | + |
|         | 868    | Dark   | 321 | 10.1[c] | 51  | + |
|         | 979    | Dark   | 390 | 12.1[c] | 62  | + |

[d]There were no nodules on the roots of pigeonpea plants inoculated with strain 748.
[e]The treatment mean differs from the wild-type control at a level of significance of 5%

In all, two nodulation mutants and nine nitrogen fixation mutants were isolated. The phenotypes of these mutants are discussed in detail below and are summarized in Table.

TABLE 2. **Symbiotic phenotypes of NC92 mutants on three host plants**

| NC92       | Groundnut | | | Pigeonpea | | | Siratro | | |
|------------|-----|-----|-----|-----|-----|-----|-----|-----|-----|
| strain no. | Nod | Fix | Ara | Nod | Fix | Ara | Nod | Fix | Ara |
| NC92       | +   | +   | +   | +   | +   | +   | +   | +   | +   |
| 21         | -   |     |     | -   |     |     | -   |     |     |
| 639        | +   | -   | -   | +   | -   | -   | +   | -   | -   |
| 807        | +   | -   | -   | +   | -   | -   | +   | -   | -   |
| 302        | +   | -   | +   | +   | -   | +   | +   | -   | +   |
| 727        | +   | -   | +   | +   | +   | +   | +   | +   | +   |
| 284        | +   | ±   | +   | +   | -   | +   | +   | ±   | +   |
| 979        | +   | ±   | +   | +   | -   | +   | +   | ±   | +   |
| 748        | +   | +   | +   | -   |     |     | +   | -   | -   |
| 831        | +   | ±   | +   | +   | ±   | +   | +   | ±   | +   |
| 853        | +   | ±   | +   | +   | ±   | +   | +   | ±   | +   |
| 868        | +   | ±   | +   | +   | ±   | +   | +   | ±   | +   |

## Physical analysis of NC92 Tn5 mutants.

DNA hybridization is used to determine whether the NC92 Nod⁻ and Fix⁻ mutants contain Tn5 sequences. EcoRI-digested genomic DNA from each mutant strain and from the wild type strain, NC92, is subjected to Southern blot analysis and probed with [32]p-labeled pGS9 or pACTC184 DNA. In 7 of the 11 mutants a single pGS9-hybridizing EcoRI fragment was observed, and no hybridization was detected to pACYC184. Since pACYC184 constitutes the region of the pGS9 vector that surrounds Tn5 (64), this result indicates that these strains contain a single Tn5 insertion and do not contain integrated vector sequences. The insertions were in different EcoRI fragments in each case.

Two of the mutants, no. 21 and 748, showed two pGS9-hybridizing EcoRI fragments. These fragments also hybridized to pACYC 184, demonstrating that these two mutants contain integrated vector sequences. The remaining two mutants, no. 831 and 979, showed no hybridizing bands with either probe, indicating that they must be spontaneous kanamycin-resistant mutants.

These same Southern blots, or replicas, are reprobed with [32]p-labeled DNA of subclones of known heterologous symbiotic genes in an attempt to correlate the observed NC92 mutant phenotypes with Tn5 (or vector) insertions in the vicinity of homologous NC92 DNA seqences. These would be detected by observing changes in the electrophoretic mobilities of the homologous NC92 restriction fragments (s). The hybridization probes used and the sizes of the hybridizing NC92 restriction fragments are listed in the Experimental Section.

Strain NC92 no. 21 was found to contain an insertion in DNA that is homologous to a 5.2-kilobase (kb) XhoI fragment which contains the common nodulation genes (nodABCD) of Bradyrhizobium sp. (Parasponia) strain Rp501 (46). However, none of the other mutants contained insertions in NC92 restriction fragments that are homologous to Bradyrhizobium nifH, nifD, nifK, fixA, fixB, fixC, a nifA-like gene, nifB, nifS, ntrB, ntrC, glnA, or glnII.

## Symbiotic phenotype of spontaneous kanamycin-resistant mutants.

Since 2 of the 11 NC92 symbiotic mutants were found to be spontaneous kanamycin-resistant mutants (a frequency of 18%), the frequency of isolation of such spontaneous mutants was checked by plating serial dilutions of a log-phase culture of wild-type NC92 on minimal sucrose plates with and without the addition of kanamycin and was found to be 1 in $5 \times 10^6$. Four of these spontaneous Km^r mutants were checked for their symbiotic phenotype on pigeonpea; one was Fix^+, two were Fix⁻ ARA⁻. Thus, there seems to be a link between spontaneous kanamycin resistance and symbiotic ineffectiveness in this strain.

## Growth characteristics of NC92 Nod⁻ and Fix⁻ mutants.

The NC92 kanamycin-resistant transconjugants were originally selected on minimal medium, thereby precluding the isolation of auxotrophic mutants. Nevertheless, two mutants do show altered growth properties. Mutant no. 868 takes 8 days to form a 1-mm-diameter colony on a plate when grown at 30°C (2 days longer than NC92 or the other Tn5-containing derivatives). Mutant no. 284 is deficient in growth in liquid media; although it will form colonies in the usual 6 days on TY, YM, and minimal plates, it will not grow at all in TY broth and only very slowly in YM broth; no. 284 takes about 7 days to saturate 5 ml of YM broth after the inoculation of a single colony, compared with about 3 days for NC92 and the other Tn5-containing mutants.

## Nitrogen fixation (Fix⁻) mutant of NC92.

The usual criterion used to identify Fix⁻ Rhizobium mutants is the acetylene reduction assay. Mutants which induce nodules but are unable to reduce acetylene in symbiotic association with their host plant are deemed to be Nod^+ Fix⁻ (49). However, as described below, several of the mutants isolated in this invention form symbiotic associations in which the host plant clearly receives little or no symbiotically fixed

5

nitrogen, yet acetylene reduction assays consistently give positive results.

Therefore, in this invention, the criterion used to assign a Fix⁻ phenotype is the relative amount of nitrogen fixed.

This is calculated as described in <u>Materials</u> <u>and</u> <u>Methods</u> by comparing the level of combined nitrogen present in plants nodulated by a particular mutant with that present in plants nodulated by the wild-type strain, NC92 (100% relative nitrogen fixation), and by the two mutants, no. 639 and 807, that showed no detectable acetylene reduction activity (0% relative nitrogen fixation).

In the following discussions the Fix phenotype of strains have been designated on the basis of their relative nitrogen fixation as follows: less than 25%, Fix⁻; between 25% and 75%, Fix±; and above 75% (or not statistically different from NC92), Fix⁺. Acetylene reduction activity, in contrast, is treated as a secondary property of Fix⁻ mutants (Ara phenotype). Since there can be a twofold variation in acetylene reduction activity from plant to plant, and since the data are not normalized to nodule weight, these data are only interpreted qualitatively; positive acetylene reduction activity (Ara⁺) is defined as being an average over all experiments of greater than 30% of wild-type NC92 activity and negative activity (Ara⁻) as being less than 3% of wild-type activity. Actual mean valuess for wild-type NC92 ranged from 4 to 23, 1 to 8, and 2 to 10 umol of $C_2H_2$ per plant per h for groundnut, pigeonpea, and siratro, respectively.

The data on shoot weight and combined nitrogen presented in Table 1 were gathered during a single-pot trial conducted at MGH. The qualitative acetylene reduction activities and the inferred symbiotic phenotypes described below and summarized in Table 2 are based on this trial and on replicative trials conducted at two different locations.

Three mutants formed Fix⁻ symbioses with all three host plants tested. The nodules induced by two of these mutants, no. 639 and 807, also failed to reduce acetylene. However, it was found that nodules induced by strain no. 302 showed levels of acetylene reduction activity comparable to those of nodules induced by wild-type NC92 on all three host plants (an average of 140, 50 and 78% of wild-type activity on groundnut, pigeonpea, and siratro, respectively), even through the relative amount of nitrogen fixed was less than 15% (Table 1).

Three mutants, no. 831, 853, and 868, showed a partially effective phenotype on all three host plants; the relative amount of nitrogen fixed fell between 43 and 64% of that fixed by wild-type NC92. Plants of all three host species inoculated with these strains remained pale green for 5 to 10 days after NC92-inoculated plants became dark green. The plants did subsequently become dark green and showed wild-type levels of acetylene reduction activity at the end of the trial, although, as other results in this application demonstrate, this does not necessarily indicate that dinitrogen gas is being fixed at wild-type levels.

Possible explanations for these partially effective phenotypes include delayed nodulation, delayed onset of nitrogen fixation, reduced efficiency of nitrogenase for dinitrogen reduction, or a lowered bacteroid concentration. The first two possibilities were investigated further by using siratro plants grown in seedling agar tubes to that the onset of nodulation could easily by monitored, and repeated, nondestructive acetylene reduction activity assays could be carried out. In two independent trails, with 10 tubes (1 plant per tube) per treatment, neither mutant no 853 nor mutant no 868 showed any significant delay in the onset of nodulation or of nitrogen fixation as determined by acetylene reduction activity, on siratro. In contrast, mutant no 831 showed a 4-to 7-day delay in nodulation and a possible further delay of several days in the onset of nitrogen fixation.

The remaining three mutants show host specific nitrogen fixation phenotypes. Mutants no. 284 and 979 are Nod⁺ Fix⁻ on pigeonpea, resulting in pale green plants with 6 and 11% relative amounts of fixed nitrogen, respectively (Table 1). In contrast, these two strains are partially effective (40 to 70% relative fixed nitrogen) on siratro and on groundnut. Acetylene reduction activity by both strains was detectable on all three hosts (an average of 31 to 185 % of the wild type). Mutant no. 727 exhibits a different host-specific phenotype. It is Nod⁺ Fix⁺ on pigeonpea and siratro (on siratro its relative nitrogen fixation was only 74%, but this was not statistically different from the wild-type control) but clearly Nod⁺ Fix⁻ on groundnut. However, mutant no 727 does exhibit considerable acetylene reduction activity in symbiosis with groundnut (an average of 60% of wild-type levels).

## Nodulation (Nod⁻) mutants of NC92.

Two NC92 nodulation mutants were identified. Mutant no. 748 was initially identified as being Nod⁺ Fix⁻ on siratro. It was subsequently found that this mutant does not elicit any nodules on the roots of pigeonpea, whereas it elicits fully effective, nitrogen-fixing nodules on groundnut. These three distinct host-specific symbiotic phenotypes of mutant no. 748 have been confirmed in several experiments.

The discovery that a single mutant could exhibit three different host-specific symbiotic phenotypes was unexpected. It was hypothesized that strain no. 748 might be defective in some function which is required for the root hair (pigeonpea and siratro [37, 57]) but not for the crack entry (groundnut [17]) mode of infection. Therefore, ineffective, mutant no. 748-induced siratro nodules were plastic embedded and sectioned for light microscopy to examine the nodule structure more closely. There were no darkly staining bacteroid packed cells, and these "empty" nodules had central (occasionally branched) vascular bundles, rather than the peripheral distribution of vascular bundles observed in wild-type siratro nodules (55). Occasional dark-staining bodies were observed in peripheral cortical cells, which could indicate limited bacterial invasion and would explain why it was possible to isolate some bacteria from these nodules. Mutant no. 748 does induce normal root hair curling and the formation of typical "shepherd's crooks" on siratro, a characteristic first step in the root hair infection pathway. This is consistent with no. 748 having a mutation in a nodulation function other than the common nodulation genes (nod ABCD) (40).

Mutant no. 21 does not induce nodules on any of the three host plants tested. This result has been repeated in seedling agar tubes (siratro) and in growth pouch (pigeonpea) experiments at two different locations. In contrast to mutant no. 748, no. 21 does not induce root hair curling on siratro. These rsults suggested that mutant no. 21 might have a lesion in one of the so-called common nodulation genes (nodABCD), which are both structurally and functionally conserved in a number of Rhizobium and Bradyrhizobium species and appear to be essential for the induction of nodules on all Rhizobium host plants, including the nonlegume Parasponia.

Therefore it was tried to genetically complement mutant no. 21 with plasmid pRmSL26, which carries the common nodulation genes cloned from Rhizobium meliloti (43). Plasmid pRmSL26 was introduced into mutant no. 21 by bacterial conjugation, and the resultant transconjugants were inoculated onto siratro. They were able to elicit nodules with high acetylene reduction activity on siratro, whereas no nodules formed on no. 21-inoculated plants. Minocycline-resistant colonies were isolated from these nodules (minocycline is a tetracycline analog which was found to have greater efficacy than tetracycline in selecting against wild-type NC92) and were able to elicit nodules on both siratro and pigeonpea. Southern blot analysis confirmed that these isolates did contain pRmSL26 sequences and were not revertants of the original mutant. Complementation of mutant no. 21 has not been tested on groundnut.

Since mutant no. 748, which was originally identified as being Fix⁻ ARA⁻ on siratro, induced uninvaded siratro nodules, siratro nodules induced by the two non host-specific, Fix⁻ ARA⁻ mutants, no. 639 and 807, were also sectioned and examined under the light microscope. In both cases the nodules contained bacteria-filled cells, showing that these two mutants are not infection mutants on siratro. Mutant no. 639-induced nodules were indistinguishable from wild typeinduced nodules; in contrast, mutant no. 807-induced nodules contained many fewer infected cells in the central, bacteroid-containing zone, and the uninfected cells showed the accumulation of large starch grains that is characteristic of ineffective nodules.

**Detection of potential cross-contamination in the pot trials.**

The major drawback of testing symbiotic mutants in open pot trials is the possibility of cross-contamination by strains from adjacent pots. Indeed, nodules were observed on the secondary roots of uninoculated control plants, However, several arguments can be advanced to suggest that cross-contamination did not occur in inoculated pots. First, nodules did not form on the roots of plants inoculated with non-nodulating mutants of NC92 (no. 21, and no. 748 on pigeonpea), suggesting that an existing rhizobial population in the growth medium competes successfully with and prevents establishment of contaminating rhizobia. Second, in one trial (Table 1), bacteria from nodules induced on one siratro and one pigeonpea plant for each treatment were isolated. All of the nodules from each treatment, including the later-formed nodules on the secondary roots, were pooled, and bacteria were isolated. These isolations were first streaked onto Congo red-containing YM plates and subsequently replica plated onto Congo red YM kanamycin plates. In all cases, all bacteria isolated were Km$^r$, indicating that no reversion of the mutants had occurred by excision of Tn5.

However, there still remained the possiblity of cross-contamination by the other Km$^r$ strains. Therefore, genomic DNA was prepared from nodule isolates from plants inoculated with mutants no. 284, 727, 831, and 979 as well as from isolates from uninoculated plants. This DNA was digested with EcoRI, run on a 0.6% agarose gel, transferred to GeneScreen filters, and hybridized with $^{32}$p-labeled pGS9 DNA. This analysis showed first that all nodules were formed by NC92 and its derivatives (as judged by EcoRI genomic fingerprints) and second that each isolate showed the pattern of pGS9 hybridization expected of the parent inoculum; that is, strains no. 284 and 727 showed a single hybridizing band of 18 and 9 kb, respectively, whereas no. 831 and 979, despite being Km$^r$, did not contain any pGS9-hybridizing bands at all. A single hybridizing band could be detected in each of the uninoculated controls.

These lines of evidence, taken together, argue strongly against the possiblilty of cross-contamination of strains in pots with an inoculated Bradyrhizobium strain and therefore uphold the validity of the data presented in Table 1.

## DISCUSSION

The suicide vector pGS9 was used to carry out random Tn5 mutagenesis of a slow-growing Bradyrhizobium strain of the cowpea cross-inoculation group. Nodulation and nitrogen fixation mutants were successfully isolated at frequencies comparable to those obtained by other workers (16, 49).

However, physical analysis of the mutants revealed two classes of mutants that do not contain simple Tn5 insertions. Of the 11 NC92 mutants, two (no. 21 and 748) contain additional pGS9 vector sequences, and two (no. 831 and 979) are spontaneous kanamycin-resistant mutants. The remaining seven mutants do contain single Tn5 insertions. However, since it was only possible to correlate the phenotype of one of the mutants (no. 21) with an insertion in a known symbiotic locus (nodABCD), further analysis is required to demonstrate conclusively that the observed symbiotic phenotypes are caused by the Tn5 insertions.

The frequency of occurrence of spontaneous kanamycin-resistant mutants among the NC92 symbiotic mutants was surprising. The phenomenon was therefore further examined and it was found that there is a high background of isolation of spontaneous mutants (1 mutant in $10^5$ to $10^6$ kanamycin-susceptible cells, comparable to the frequency of isolation of kanamycin-resistant colonies after pGS9 mutagenesis), and a strong correlation between acquired kanamycin resistance and symbiotic defectiveness in strain NC92.

Correlations between spontaneous antibiotic resistance and reduced symbiotic effectiveness have previously been reported (52, 53), although Pankhurst (53) found that spontaneous kanamycin resistance reduced the symbiotic effectiveness of Rhizobium but not Bradyrhizobium strains. It is possible that the acquired resistance results from alterations in cell wall permeability and that these changes also disrupt bacterial communication with the plant partner. Alternatively, the observed symbiotic phenotypes could result from transposition of an endogenous insertion element, as was found in R. meliloti (61), although this hypothesis offers no explanation for the correlated acquisition of kanamycin resistance. Such spontaneous kanamycin-resistant mutants could be avoided if it were possible to select for the presence of Tn5 by coselectionn with kanamycin and streptomycin, thus exploiting the additional Tn5-encoded strepto mycin resistance gene which is expressed in Rhizobium species (22, 56, 58, 65). Unfortunately, the endogenous streptomycin resistance of strain NC92 is too high (resistant to 500 ug/ml) to make this approach feasible, and this may be a common problem with other Bradyrhizobium cowpea group strains (38).

The symbiotic mutants which have been isolated herein exhibit several novel phenotypes. Among the Fix$^-$ mutants, only two mutants (no. 639 and 807) exhibit a conventional Fix$^-$ phenotype; the inability of these two mutants to supply their plant hosts with fixed nitrogen is attributable to the absence of active nitrogenase enzyme, as demonstrated by the failure of nodules elicited by these mutants to reduce acetylene. Light microscopic examination of no. 639-and 807-elicited siratro nodules did not reveal any obvious defects in bacterial invasion. It was therefore particularly surprising that even these two mutants did not appear to contain insertions in the nitrogenase structural genes nifHDK, or in any other known symbiotic genes, and that no such mutants were isolated.

Three mutants (no. 831, 853, 868) show partially effective phenotypes on all three host plants. One of these (no. 831, which is one of the spontaneous kanamycin-resistant mutants) exhibits a small nodulation delay in siratro. If nodulation were also delayed on the other two host plants, this could perhaps account for the reduced effectiveness of this strain. The reason for the reduced effectiveness of the other two strains is not known.

Another mutant (no 302) is clearly Fix$^-$ on all three host plants and yet exhibits considerable acetylene reduction activity, demonstrating the presence of nitrogenase enzyme with at least partial activity. The only well-characterized precedent for such a phenotype lies with the free-living, nitrogen-fixing species Klebsiella pneumoniae; K. pneumoniae strains with a mutation at the nifV locus fail to process the nitrogenase iron-molybdenum cofactor correctly, producing an enzyme which is able to reduce acetylene but not nitrogen (31, 48). A consequence of this incomplete processing is that the acetylene reduction activity is not susceptible to competitive inhibition by dinitrogen, unlike the wild-type enzyme.

Dinitrogen inhibition of acetylene reduction activity has not been examined in the present mutants. Moreover, no nifV-like gene has yet been identified in any Rhizobium or Bradyrhizobium species, and so the possibility that mutant no. 302 is defective in a nifV-like gene could not be ruled out by DNA hybridization. There are also some reports of B. japonicum strains with similar phenotypes; soybean (Glycine max) plants inoculated with these strains are yellow and nitrogen starved, but the nodules exhibit considerable acetylene reduction activity. One such strain contains an engineered deletion in a gene that is homologous to the K. pneumoniae nifS gene, a gene encoding a factor involved in molybdenum cofactor processing (32A). However, Southern blot analysis ruled out the possibility that any of the NC92 mutants contained insertions in an NC92 nifS-homologous gene. another ineffective B. japonicum mutant which retains acetylene reduction activity has been reported (75). This mutant lacks glutamate-oxaloacetate amino transferase and may be defective in transferring fixed nitrogen to the plant. Thus, possible explanations for this phenotype (Fix$^-$ Ara$^+$) include defects in enzyme processing or in molecular transport.

The remaining three NC92 Fix$^-$ mutants are Fix$^-$ on only a subset of the host plants tested. These mutants are no. 284 and 979, which are Fix$^-$ on pigeonpea but Fix± on siratro and groundnut, and no. 727, which is Fig$^-$ on groundnut but Fix$^+$ on siratro and pigeonpea. In each case it does not seem possible to attribute the Fix$^-$ or Fix± phenotype directly to reduce nitrogenase activity, since each mutant exhibited an average of at least 31% of wild-type NC92 acetylene reduction activity, even on the host plants that received less than 25% relative fixed nitrogen. It is curious that mutants no. 979 lacks any Tn5 insertion at all. The one difference between no. 284 and 979 both show such similar phenotypes when no. 284 and 979 is that only no 284 shows impairment of growth in liquid media.

Such bacterial host-specific fixation mutants, which are termed Hsf mutants by analogy to Hsn (host specific nodulation) mutants (40), have not previously been reported. This may simply be because Rhizobium mutants are not usually screened for symbiotic effectiveness on more than one host plant. The phenomenon of host-specific fixation is well known among natural isolates of both Rhizobium and Bradyrhizobium species (27), and Appelbaum et al. were recently able to transfer cultivar-specific nitrogen fixation ability between strains of Rhizobium fredii, the fast-growing symbiont of soyben, by transferring the indigenous symbiotic plasmids (1). Moreover, plant mutants that exhibit altered strain specificity for the formation of effective nodules have been isolated (12). The NC92 mutants have not been tested on sufficient cultivars to determine whether the Hsf phenotypes lie at the host cultivar of species level.

It is possible that host-specific fixation is similar to host-specific nodulation in that a specific negative interaction occurs at an early stage in the nodulation pathway, but that the barrier lies in nodule invasion rather than nodule induction. However, light microscopic examination of ineffective siratro nodules induced by a B. japonicum strain, USDA 122, which forms a fully effective symbiosis with soybean, revealed that these siratro nodules are indeed invaded by strain USDA 122 (K. Wilson, unpublished results). Thus, the host-specific failure to fix nitrogen in this case must be due to some postinfection breakdown in the interaction between the plant and the bacterium. This is almost certainly also true for the NC92 mutants, since each exhibits significant nodule acetylene reduction activity (in contrast to the siratro nodules induced by USDA 122). Such Hsf defects could lie in specific signaling between the symbiotic partners or in some aspect of nodule physiology. For example, the pathway of infection (17) and the structure of the bacteroids (66) are both very unusual in groundnut, and may require unique capabilities in the bacterium which are absent in mutant no. 727. Another possibility is that different host plants may supply different carbon substrates to the bacteroids, and these NC92 mutants might be deficient in the utilization of host-specific carbon sources.

It has also been shown that a reduction in the levels of exported, fixed nitrogen can lead to a failure by the plant to induce the enzymes necessary for nitrogen assimilation and to early senescence of the nodules (2,3), and this effect might be greater in certain host species than in others.

The identification of such a high proportion of Fix$^-$ Ara$^+$ mutants in strain NC92 may be a consequence of the different modes of regulation of nif genes between fast-and slow-growing rhizobia. It has not been possible to demonstrate ex planta acetylene reduction activity in fast-growing Rhizobium strains, whereas such ex planta activity is well documented in Bradyrhizobium strains (42, 47, 54). Therefore it could be speculated that fast-growing Rhizobium species might exhibit more stringent regulatory mechanisms which prevent the expression of nitrogenase when any of a number of symbiotic genes are mutated, whereas analogous defects in the symbiotic genes of Bradyrhizobium strains would not shut down the induction of nitrogenase.

In addition to the nine Fix mutants, also described herein is the isolation of two Nod$^-$ mutants. Mutant no. 21 fails to elicit nodules on all three host plants. Genetic complementation and DNA hybridization studies indicate that it carries a mutation in the common nodulation genes. The fact that mutant no. 21 fails to elicit nodules on groundnut, a crack entry host, as well as on pigeonpea and siratro, which are both

9

infected via the root hairs, confirms the findings of others (7, 51, 46A) that common nod genes are required for both modes of infection, and therefore that they are not simply genes for the induction of root hair curling or infection thread formation.

The second nodulation mutant, no. 748, is a host-specific nodulation mutant. It is known that both fast- and slow-growing rhizobia which exhibit a broad host range carry separate recognition factors for at least some of their hosts; certain genes have been identified by the transfer of partial host range to other Rhizobium species (5, 6, 11), and a mutant Bradyrhizobium sp. (Parasponia) strain has been constructed that fails to elicit nodules on the nonlegume host Parasponia sp. but is still able to elicit Fix[+] nodules on two legume hosts, siratro and cowpea (Vigna unguiculata) (46A).

Mutant no. 748 is particularly interesting because it exhibits a different mutant phenotype on each of three host plants. On pigeonpea it fails to induce any nodules at all, suggesting that it may lack a specific "pigeonpea factor." It does induce nodules on siratro, but the nodules are devoid of bacteria and have abnormal, centralized vasculature. Rhizobium mutants which induce such empty nodules have recently been identified in the fast-growing Rhizobium species (24, 26, 71), and mutant no. 748 demonstrates that the processes of nodule induction and nodule invasion are also separable in the slow-growing Bradyrhizobium species. The structure of these empty siratro nodules resembles that of Phaseolus vulgaris (common bean) nodules induced by noninfective mutants of Rhizobium phaseoli (71) and may therefore be characteristic of uninvaded, determinate nodules. On groundnut, the crack entry host, no. 748 shows no defect in nodulation or nitrogen fixation. Thus, actual infection by mutant no. 748 may be limited to crack entry hosts, making no. 748 a root hair infection mutant, although it is clear that infection by no. 748 should be tested on other hosts, particularly on the crack-entry Stylosanthes species (18), before any definite conclusions can be drawn. This hypothesis does not fully explain the difference in the interaction of no. 748 with the two root hair infection hosts, siratro and pigeonpea. One possibility is that siratro and pigeonpea have very different sensitivities for nodulation factors, as demonstrated by Bauer et al. (8) for soybean and cowpea, such that the absence of a certain factor affects the two hosts differentially. It is also not yet certain whether these different phenotypes are attributable to a single or two multiple mutations in mutation no. 748. However, this mutant does clearly demonstrate that different host plants do have different qualitative or quantitative requirements for nodulation factors.

In this description has been demonstrated the utility of random transposon mutagenesis followed by careful screening in the isolation of novel symbiotic Bradyrhizobium mutants. Several of these mutants do not have a direct phenotypic counterpart among known symbiotic mutants of the fast-growing Rhizobium species, and only one (no. 21) was shown to carry an insertion in a DNA sequence that shares homology with a known Rhizobium symbiotic gene.

## USES

The Tn5 mutagenized mutants of the invention can be used as sources for the isolation of the wild type genes involved in providing the original phenotype which has been mutated. For example, a mutant which has lost the ability to nodulate a given first host can be used to isolate the nodulation gene for that host. This is done by using the km[R] selectable marker, which is part of the Tn5 insert, as a tag to isolate a molecular clone of the Tn5 insert and adjacent Bradyrhizobium genetic sequences. These adjacent Bradyrhizobium DNA sequences are then separated out and used as probes for isolating the complete wild-type gene from an appropriate gene library. Once such a host range gene has been isolated it can be introduced into other rhizobial species, which are previously unable to nodulate and/or fix nitrogen on that particular host, and can thereby expand the host range of the rhizobial species. Thus, the utility of an efficient inoculant rhizobial strain can be enhanced by expanding its host range through the use of such cloned Bradyrhozobium host range genes.

## DEPOSITS

Strains 727 and 748 were deposited prior to the U.S. filing data at the American Type Culture Collection, Rockville, Maryland, under the auspices of the Budapest Treaty. The accession numbers are as follows:

Bradyrhizobium sp. (Arachis) Mutant 748: ATCC No. 53612
Bradyrhizobium sp. (Arachis) Mutant 727: ATCC No. 53611

MATERIALS AND METHODS

**Bacterial strains and plasmids.**

Bradyrhizobium sp. (Arachis) strain NC92 is an isolate from groundnut provided by G. Elkan (North Carolina State University). The bacterial strains and plasmids used in this study are listed in Table 3.

## TABLE 3. Plasmids Used in the Experiments

| Plasmid | Relevant Characteristics | Source or reference | Probe | NC92[a] |
|---|---|---|---|---|
| pGS9 | Tn5 suicide vector | 64 | | |
| pACYC184 | Probe for pGS9 vector sequences | 19 | | |
| pRK2013 | Helper plasmid for pRmSL26 conjugation | 23 | | |
| pRmSL26 | Cosmid clone carrying R. meliloti nodABCD | 43 | | |
| Hybridization probes | | | | |
| pRH232D | Bradyrhizobium sp. (Vigna) nifH in pBR322 | 35 | 1-kb SmaI-HindIII fragment | 3.5 |
| pBN187 | Bradyrhizobium sp. (Parasponia) 1.5-kb EcoRI fragment containing N-terminal 600 base pairs of nifD in pUC13 | B.T. Nixon | Plasmid | 2.5 |
| pRN231 | Bradyrhizobium sp. (Vigna) nifK in pBR322 | 35 | 2.6-kb BamHI-HindIII fragment | 5.0, 14.0 |
| pBJ152 | B. japonicum nifA-like gene in pBR328 | B.K. Chelm | Plasmid | 15.0 |
| pBN384 | Bradyrhizobium sp. (Parasponia) nifB in pUC 13 | B.T. Nixon | Plamis | 7.0 |
| pBJ135 | B. japonicum nifS in pBR328 | B.K. Chelm | Plasmid | 7.0, 4.0 |
| pBJ150 | B. japonicum fixA and partial nifA-like gene in pBR328 | B.K. Chelm | 2.6-kb XhoI-EcoRI fixA fragment | 15.0 |
| pBN259 | Bradyrhizobium sp. (Parasponia) fixB in pUC13 | B.T. Nixon | Plasmid | 3.5 |
| pBN408 | Bradyrhizobium sp. (Parasponia) nifH fixB fixC in pUC13 | B.T. Nixon | 1.1-kb fixC PstI fragment | 5.0 |
| pBN176 | Bradyrhizobium sp. (Parasponia) ntrB in pUC13 | B.T. Nixon | 0.9-kb EcoRI fragment | 11.0 |
| pBN386 | Bradyrhizobium sp. (Parasponia) ntrC C-terminal 1 kb in SP6 | B.T. Nixon | Plasmid | 10.0, 3.5, 2.5 |
| pPRC6 | Bradyrhizobium sp. (Parasponia) nodABCD genes in pLAFR1 | 46 | 5.2-kb XhoI fragment | 11.5 |
| pBJ53A | B. japonicum glnA in pUC8 | 15 | Plasmid | 14.0 |
| pBJ196A | B. japonicum glnII in pBR322 | 14 | Plasmid | 9.0 |

[a] Size of hybridizing NC92 EcoRI fragment(s) in kilobase pairs.

**Bacterial media.**

LB(49), TY(50), YM(72), and Sherwood minimal medium (67) have been described. Nutrient agar was 5 g of peptone, 5 g of NaCl, 1.5 g of beef extract, and 1.5 g of yeast extract per liter. Minimal sucrose medium was 2% sucrose, 0.05 g of $K_2HPO_4$, 0.15 g of $KH_2PO_4$, 0.2 g of $MgSO_4$, 0.2 mg of $Na_2MoO_4$, 6.6 mg of $FeCl_3$, 0.5 g of sodium glutamate, 1 g of $CaCO_3$ per liter, and 0.025% bromothymol blue. Media were solidified with 1.5% agar (Difco Laboratories, Detroit, Mich.) as required. Congo red was added to YM plates to a final concentration of 25 ug/liter (72) to facilitate the detection of potential contaminants (39). LB medium was supplemented with kanamycin (20 ug/ml) or tetracycline (10 ug/ml) for the growth of Escherichia coli strains when appropriate. YM and minimal sucrose media were supplemented with Kanamycin (100 ug/ml), Sherwood medium was supplemented with tetracycline (150 ug/ml), and TY was supplemented with the tetracycline analog minocycline (10 ug/ml) (13) for the growth of Bradyrhizobium transconjugants when appropriate.

**Isolation of symbiotic mutants.**

Bacterial matings between E. coli strain WA803 (64) and Bradyrhizobium sp. (Arachis) strain NC92 were carried out by coincubation of the two strains on nonselective TY plates at 28°C for 24 hr. Km$^r$ NC92 transconjugants were then selected by plating the resuspended mating mixture on minimal sucrose (kanamycin) plates and were purified three times through single colonies; 923 Km$^r$ transconjugants were serially screened for their symbiotic phenotype on goundnut plants grown in pots in the greenhouse and on siratro plants grown individually in tubes in the growth chamber (two tubes per transconjugant). Putative symbiotic mutants were further tested in replicated trials conducted at both locations.

**Preparation of inoculum.**

The inoculum was prepared by using 12 g of gamma-irradiated peat packets (70) obtained from Agricultural Laboratories Pty. Ltd., Australia. Log-phase cultures of Km$^r$ NC92 transconjugants were grown in YM broth at 28°C (3 to 4 days of growth), and 10 ml of this broth was injected into a fresh peat packet; the broth and peat were thoroughly mixed and incubated for 10 days at room temperature. During this time the Bradyrhizobium population grows to $10^8$ to $10^9$ cells per g of peat. The peak packets were then stored at 4°C until use, when the peat was diluted in sterile water to $10^6$ cells per ml; 1 ml of inoculum was used per seed. This inoculum was used for all greenhouse and growth chamber trials. Alternatively, frozen inoculum was prepared using a modification of the method of Bhuvaneswari et al. (9). Strains were grown to the midlog phase in TY broth (optical density at 620 nm of 0.4), 5 x $10^9$ cells were pelleted and suspended in 5 ml of 1:10 phosphate-buffered saline (8 g of NaCl, 0.2 g of $KH_2PO_4$, 2.9 g of $Na_2HPO_4$, and 0.2 g of KCl per liter) plus 15% glycerol, and 0.5-ml samples were frozen at -70°C. These were subsequently diluted 10x or 100x in sterile water for use as inoculum with $10^8$ or $10^7$ cells per ml, respectively.

**Plant culture and growth measurements.**

Groundnut cultivar Robut 33-1, pigeonpea cultivar ICP-1, and siratro were all obtained from ICRISAT. Additional pigeonpea, cultivar T-21, was obtained from Niftal, Paia, Hawaii, and additional siratro seed was from Wright Stephenson Co. (Australia) Pty. Ltd., New South Wales, Australia. Seeds were sterilized by immersion in concentrated sulfuric acid for 12 min (siratro) or 0.2% $HgCl_2$ for 3 min (groundnut and pigeonpea), followed by six rinses in sterile water.

**Greenhouse trials.**

Plants were grown in a 2:1 (vol/vol) mixture of sand and vermiculite in 6-in. (ca. 18-cm)-diameter pots. Two plants were grown per pot. Plants were watered with nitrogen-free nutrient solution, either Broughton solution (10) or Bergersen solution (29). Greenhouse trials at MGH were conducted at 24°C with a 12-h day length. Higher temperatures were used. at the other location, where the ambient temperature could exceed 40°C, and no additional lighting was provided. At both locations the surface of the pots was covered with sterilized gravel after the plants had emerged to reduce splashing and possible cross-contamination among pots. At one location the pots were additionally fitted with watering tubes (P.T.C. Nambiar and P.J. Dar, Proceedings of the International Workshop on Groundnuts, ICRISAT, Patenacheru, India, 1980), whereas at the other the pots were enclosed in open-ended plastic bags to further reduce this problem (35).

Plants were harvested after about 40 days of growth. The shoots were dried for 2 days at 60°C and weighed. The dried shoots were milled with a Ud Cyclone Sample Mill (Ud Corporation, Boulder, Colo) and an approximately 15-mg subsample was analyzed for percentage combined nitrogen on a Carlo Erba automated nitrogen analyzer (ANA 1500; Carlo Erba Strumentazione, Milan, Italy). The relative amount of nitrogen fixed ($\underline{RN}$) was calculated by using the formula $\underline{RN}$-(mean total $\underline{N}_{mutant}$ -mean total $\underline{N}_{639/807}$)/mean total $\underline{N}_{NC92}$-mean total $\underline{N}_{639/807}$), where $\underline{N}$ is the total nitrogen fixed, and 639 and 807 are two NC92 derivatives that showed no detectable nitrogenase activity, as judged by the acetylene reduction assay, in symbiosis with any of the three host plants tested. For acetylene reduction assays, detached roots were incubated for 2 h in 10% acetylene in 125-ml Erlenmyer flasks sealed with a no. 49 Suba Seal (Gallenkamp, United Kingdom). After 2 h a 5-ml gas sample was removed and injected into a 10-ml Vacutainer (Becton Dickerson Labora tories, Oxnard, Calif.) for temporary storage. Gas samples were subsequently analyzed for acetylene reduction activity on a Sigma 3B gas chromatogrph (Perkin-Elmer Corp., Nowalk, Conn.).

In all pot trials each treatment was replicated from three to five times. The pots were arranged in a randomized complete block or in a split plot design, and the results were analyzed statistically by using analysis of variance, by the Dunnett test for comparing a control mean to each other group mean (74), and by calculating the least significant difference (68).

**Growth chamber trials.**

Siratro plants were also grown in seedling agar tubes (72), and pigeonpeas were grown in growth pouches (9). Growth pouches were watered with Broughton or Bergerson solution. Growth cabinets were maintain at 25°C with 16-h day length. Siratro acetylene reduction activity was measured on intact plants in tubes as described previously (49).

**Isolation of bacteria from nodules.**

Nodules were immersed in 0.2% $MgCl_2$ for 60 s, rinsed six times in sterile distilled water, and squashed in 0.1 ml of sterile water. Nodule isolations were first streaked on nonselective YM plates and then streaked or replica plated on selective media as appropriate.

**Complementation of mutant NC92 no. 21.**

Three-way matings were conducted as described previously (60) among NC92 no. 21, E. coli HB101 containing the helper plasmid. pRK2013, and E. coli HB101 containing plasmid pRmSL26. Tc$^r$ NC92 no. 21 transconjugants were selected on Sherwood minimal plates with 150 ug of tetracycline per ml.

## Microscopy.

Siratro nodules were fixed and prepared for light microscopy as described previously (33).

## DNA biochemistry.

Bradyrhizobium genomic DNA was prepared as follows. A 1.5-ml samples of a saturated culture grown in TY or YM at 30°C was pelleted in a 1.5-ml microcentrifuge tube, suspended in 600 ul of 0.5% sodium dodecyl sulfate-100 ug of proteinase K (Boehringer Mannheim Biochemicals, Indianapolis, Ind.) per ml in TE (10 mM Tris, 1 mM EDTA, pH 8.0) and incubated for 1 h at 37°C. The solution was then brought to approximately 0.7 M NaCl by the addition of 100 ul of 5 M NaCl before the addition of 80 ul of 10% hexadecyltrimethylammonium bromide in 0.7 M NaCl. The tubes were incubated for 10 min at 65°C and then extracted once with 0.7 ml of 24:1 (vol/vol) chloroformisoamyl alcohol and once with 24:24:1 (vol/vol/vol) phenolchloroform-isoamyl alcohol. The supernatant was preciptated with 0.6 volume of isopropanol, the chromosomal DNA was spun down, and the pellet was washed once with 70% ethanol, dried, and suspended in 100 ul of Tris-EDTA (pH 8.0). Samples (15 ul) of this DNA were digested with 10 U of EcoRI for use on Southern blots. Small-and large-scale preparations of plasmid DNA were carried out by using the alkaline lysis procedure described previously (45). Restriction endonucleases were purchased from Boehringer Mannheim and used as described by the manufacturer. Agarose gel electrophoreses was carried out as described previously (59), and the DNA in the gel was transferred to GeneScreen filters (New England Nuclear Corp., Boston, Mass.) according to the manufacturer's instructions, except that 20 x SSC (3 M Nacl, 0.3 M sodium citrate) was used for the transfer buffer. Nick translations (45) and DNA hybridization (46) were carried out as described previously. Where necessary, DNA fragments for use as hybridization probes were isolated from low-melting-temperature agarose gels (International Biotechnologies Inc.) by melting the agarose at 70°C in the presence of 0.4 M NaCl, cooling to 37°C, and then phenol extracting twice before ethanol precipitation.

## LITERATURE CITED

1. **Appelbaum, E.R., et al.** 1985. J. Bacteriol. 163:385-388.
2. **Atkins, C.A., et al.** 1984. Plant Physiol. 76:705-710.
3. **Atkins, C.A., et al.** 1984. Planta 162:316-326.
4. **Ausubel, F.M.** 1984. P. 275-298. In R. Losick and L. Shapiro (ed.), Microbial development. Cold Spring Harbor Laboratory, Cold Spring Harbor, N.Y.
5. **Bachem, C.W.B., et al.** 1985. Mol. Gen. Genet. 199:276-278.
6. **Bassam, B.J. et al.** 1986. Mol. Gen. Genet. 203:49-57.
7. **Batut, H., et al.** 1985. p. 109-115. In H.J. Evans, P.J. Bottomley, and W.E. Newton (ed.), Nitrogen fixation research progress. Martinus Nijhoff, Dordrecht, The Netherlands.
9. **Bhuvaneswari, T.V., et al.** 1980. Plant Physiol. 66:1027-1031.
10. **Broughton, W.H., et al.** 1971. Biochem. J. 125:1075-1080.
11. **Broughton, W.J., et al.** 1986 J. Cell Biol. 102:1173-1182.
12. **Caldwell, B.E., et al.** 1977. p. 557-576. In R.W.F. Hardy and W.S. Silver (ed.), Dinitrogen fixation, section III: biology. John Wiley & Sons, Inc., New York.
13.**Cappel, R., et al.** 1971. Curr. Ther. Res. 13:27-233.
14. **Carlson, T.A., et al.** 1986. Nature (London) 322:568-570.
15. **Carlson, T.A., et al.** 1985 J. Bacteriol. 162698-703.
16. **Cen, Y., et al.** 1982. Appl. Environ. Microbiol. 43:233-236.
17. **Chandler, M.R.** 1978. J. Exp. Bot. 29:749-755.
18. **Chandler, M.R., et al.** 1982. J. Exp. Bot. 33:47-57.
19. **Chang, A.C.Y., et al.** 1978. J Bacteriol. 134:1141-1156.
20. **Dale Noel, K., et al.** 1982. J. Bacteriol. 152:485-494.
21. **Dart, P.** 1977. p. 367-472. In R.W.F. Hardy and W.S. Silver (ed.), Dinitrogen fixation, section III: biology. John Wiley & Sons, Inc., New York.

22. de Vox, G., et al. 1984. J. Bacteriol. 159:395-399.

23. Ditta, G., et al. 1980. Proc. Natl. Acad. Sci. USA 77:7347-7351.

24. Dylan, T., et al. 1980. Proc. Natl. Acad. Sci. USA 84:4403-4407.

25. Elkan, G.H. 1981. p. 191-224. In K. Giles and A. Atherly (ed.), Biology of the Rhizobiacaea. Academic Press, Inc., New York.

26. Finan, T.J., et al. 1985. Cell 40:869-877.

27. Fred, E.B., et al. 1932. 5. University of Wisconsin, Madison.

28. Fuhrmann, M., et al. 1985. Mol. Gen. Genet. 199:315-322.

29. Gibson, A.H. 1980. p. 139-184. In F.J. Bergerson (ed.), Methods for evaluating biological nitrogen fixation. John Wiley & Sons, Inc., New York.

30. Hahn, M., et al. 1984. Mol. Gen. Genet. 193:46-52.

31. Hawkes, T.R., et al. 1984. Biochem. J. 217:317-321.

32. Hennecke, H. 1981. Nature (London) 291:354-355.

32A. Hennecke, H., et al. 1987. In D.P.S. Verna and N. Brisson (ed.), Molecular Genetics of Plant-Microbe interations. Martinus Nhyoff, Dordrecht, The Netherlands.

33. Hirsch, A.M., et al. 1983. J. Bacteriol. 155:367-380.

34. Hom, S.S.M., et al. 1984. J. Bacteriol. 159:335-340.

35. Jagadish, M.N., et al. 1984 Mol. Gen. Genet. 196:290-300.

36. Jordan, D.C. 1982. Int. J. Syst. Bacteriol. 32:136-139.

37. Kapil, R.N., et al. 1971. Phytomorphology 21:192-202.

38. Kennedy, C., B. Dreyfus, and J. Brockwell. 1981. J. Gen. Microbiol. 125:233-240.

39. Kneen, B.E., et al. 1983. Appl. Env. Microbiol. 45:340-342.

40. Kondorosi, E., et al. 1984. Mol. Gen. Genet. 193:445-452.

41. Kondorosi, E., et al. 1986. Trends Biochm. Sci. 11:296-299.

42. Kurz, W.G.W., et al. 1975. Nature (London) 256:407-409.

43. Long, S.R., et al. 1982. Nature (London) 298:485-488.

44. Maier, R.J., et al. 1976. J. Bacteriol. 127:763-769.

45. Maniatis, T., et al. 1982. Cold Spring Harbor Laboratory, Cold Spring Harbor, N.Y.

46. Marvel, D.J., et al. 1985. Proc. Natl. Acad. Sci. USA 82:5841-5845.

46A. Marvel, D.J., et al. 1987. Proc. Natl. Acad. Sci. USA 84:1319-1323.

47. McComb, J.A., et al. 1975. Nature (London) 256:409-410.

48. McLean, P.A., et al. 1981. Nature (London) 292:655-656.

49. Meade, H.M., et al. 1982. J. Bacteriol. 149:114-122.

50. Miller, J.H. 1972. Cold Spring Harbor Laboratory. Cold Spring Harbor, N.Y.

51. Noti, J.F., et al. 1985. Proc. Natl. Acad. Sci. USA 82:7379-7383.

52. Pain, A.N. 1979. J. Appl. Bacteriol. 47:53-64.

53. Pankhurst, C.E. 1977. Can. J. Microbiol. 23:1026-1033.

54. Pagan, J.D., et al. 1975. Nature (London) 256:406-407.

55. Price, G.D., et al. 2985. Bot. Gaz. 145:444-451.

56. Putnoky, P., et al. 1983. Mol. Gen. Genet. 191:288-294.

57. Ridge, R.W., et al. 1986. J. Plant Physiol. 122:121-137.

58. Rostas, K., et al. 1984. Mol. Gen. Genet. 197:230-235.

59. Ruvkun, G.B., et al. 1980. Proc. Natl. Acad. Sci. USA 77:191-195.

60. Ruvkun, G.B., et al. 1980. Nature (London) 289:85-88.

61. Ruvkun, G.B., et al. 1982. J. Mol. Appl. Genet. 1:405-418.

62. Scott, K.F. 1986. Nucleic Acids Res. 14:2905-2919.

63. Scott, K.F., et al. 1983. DNA 2:141-148.

64. Selvaraj, G., et al. 1983. J. Bacteriol. 156:1292-1300.

65. Selvaraj, G., et al. 1984. J. Bacteriol. 158:580-589.

66. Sen, D., et al. 1986. J. Exp. Bot. 37:356-363.

67. Sherwood, M.T. 1970. J. Appl. Bacteriol. 33:708-713.

68. Sokal, R.R., et al. 1981. Biometry. W.H. Freeman and Co,. San Francisco.

69. Stacey, G., et al. 1982. Microbiol. 132:219-224.

70. Thompson, J.A. 1980. p. 489-534. In F.J. Bergerson (ed.), Methods for evaluating biological nitrogen fixation. John Wiley & Sons, Inc., New York.

71. Vandenbosch, et al. 1985. J. Bacteriol. 162:950-959.

72. Vincent, J.M. 1970. A manual for the practical study of root-nodule bacteria. Handbook no. 15, International Biological Programme. Blackwell, Oxford.

73. **Vincent, J.M.** 1980. In W.E. Newton and W.H. Orme-Johnson (ed.), Nitrogen fixation II: symbiotic associations and cyanobacteria. University Park Press. Baltimore.

74. **Zar, J.H.** 1974. Biostatistical analysis. Prentice-Hall Inc., Englewood Cliffs, N.J.

75. **Zlotnikov, K.M.,** et al. 1984. Doklady Akademii Nauk USSR 275:189-192.

## Claims

1. A Tn5-mutagenized rhizobial strain which is incapable of fixing nitrogen (Fix⁻) and/or of nodulating (Nod⁻) a host plant therefor.

2. The strain of claim 1 wherein said strain is Fix⁻ on only certain plant hosts therefor.

3. The strain of claim 1 wherein said strain is Nod⁻ only certain plant hosts therefor.

4. A process for preparing rhizobial strains which are Fix⁻ and/or Nod⁻, characterized by mutagenizing a rhizobial strain with Tn5 and selecting the strains having the desired characteristics.

5. A process as claimed in claim 4, characterized by selecting a Fix⁻ mutant.

6. A process as claimed in claim 4, characterized by selecting a Nod⁻ mutant.

7. A process as claimed in claim 5, characterized by selecting a mutant which is Fix⁻ on certain plant hosts only.

8. A process as claimed in claim 6, characterized by selecting a mutant being Nod⁻ with respect to certain plant hosts only.

9. The use of a mutant as claimed in anyone of claims 1 to 3 or as obtained in a process as claimed in anyone of claims 4 to 8 for isolating a complementing gene.

10. The use of a mutant as claimed in anyone of claims 1 to 3 or as obtained in a process as claimed in anyone of claims 4 to 8 for isolating a complementing gene and incorporating this gene into a plant cell.

Claims for the following Contracting State : ES

1. A process for preparing rhizobial strains which are uncapable of fixing nitrogen (Fix⁻) and/or of nodulating (Nod⁻) a host plant therefor, characterized by mutagenizing a rhizobial strain with Tn5 and selecting the strains having the desired characteristics.

2. A process as claimed in claim 1, characterized by selecting a Fix⁻ mutant.

3. A process as claimed in claim 1, characterized by selecting a Nod⁻ mutant.

4. A process as claimed in claim 2, characterized by selecting a mutant which is Fix⁻ on certain plant host only.

5. A process as claimed in claim 3, characterized by selecting a mutant being Nod⁻ with respect to certain plant hosts only.

6. The use of a mutant obtained in a process as claimed in anyone of claims 1 to 5 for isolating a complementing gene.

7. The use of a mutant obtained in a process as claimed in anyone of claims 1 to 5 for isolating a complementing gene and incorporating this gene into a plant cell.

## DOCUMENTS CONSIDERED TO BE RELEVANT

EP 88106893.6

| Category | Citation of document with indication, where appropriate, of relevant passages | Relevant to claim | CLASSIFICATION OF THE APPLICATION (Int Cl 4) |
|---|---|---|---|
| D,X | APPLIED AND ENVIRONMENTAL MICRO-BIOLOGY, vol. 43, no. 1, January 1982 (Washington DC.)<br><br>Y. CEN et al. "Transposon Mutagenesis in Rhizobia Which Can Nodulate Both Legumes and the Nonlegume Parasponia"<br>pages 233-236<br><br>* Page 235, table 2 *<br>-- | 1-8 | C 12 N 15/00<br>C 12 N 1/20<br>(C 12 N 1:20;<br>C 12 R 1:41) |
| D,X | PROCEEDINGS OF THE NATIONAL ACADEMY OF SCIENES OF THE UNITED STATES OF AMERICA, vol. 82, no. 21, November 1985 (Baltimore,USA)<br><br>J.D. NOTI et al. "Isolation and characterization of nodulation genes from Bradyrhizobium sp. (Vigna)strain IRc 78"<br>pages 7379-7383<br><br>* Abstract; page 7381, table 1 *<br>-- | 1-8 | |
| D,X | JOURNAL OF BACTERIOLOGY, vol. 163, no. 1, July 1985, (Washington DC.)<br><br>E.R. APPELBAUM et al. "Expression of Symbiotic Genes of Rhizobium japonicum USDA 191 in Other Rhizobia"<br>pages 385-388<br><br>* Totality *<br>-- | 1-8 | |

TECHNICAL FIELDS SEARCHED (Int Cl 4)

C 12 N

The present search report has been drawn up for all claims

| Place of search | Date of completion of the search | Examiner |
|---|---|---|
| VIENNA | 29-07-1988 | WOLF |

European Patent Office

# EUROPEAN SEARCH REPORT

Application number

EP 88106893.6

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| D,X | JOURNAL OF BACTERIOLOGY, vol. 159, no. 1, July 1984 (Washington DC.) S.S.M. HOM et al. "Transposon Tu 5-Induced Mutagenesis of Rhizobium japonicum Yielding a Wide Variety of Mutants" pages 335-340  \* Abstract; page 238, left column \* | 1,2,4, 5 | |
| D,X | MOLECULAR & GENERAL GENETICS, vol. 197, 1984 (Berlin, Heidelberg, New York) K. ROSTAS et al. "Transposon mutagenesis of Rhizobium japonicum" pages 230-235  \* Page 233, table 2 \* | 1,2,4, 5 | |
| X | ARCHIVES, OF MICROBIOLOGY, vol. 144, no. 2, March 1986 (Berlin, Heidelberg, New York) E. BOLTON et al. "Dicarboxylic acid transport in Rhizobium meliloti: isolation of mutants and cloning of dicarboxylic acid transport genes" pages 142-146  \* Abstract; page 143, left column; page 145, left column \* | 1,2,4, 5 | |

TECHNICAL FIELDS SEARCHED (Int Cl.4)

The present search report has been drawn up for all claims

| Place of search | Date of completion of the search | Examiner |
|---|---|---|
| VIENNA | 29-07-1988 | WOLF |

European Patent Office

# EUROPEAN SEARCH REPORT

EP 88106893.6

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,X | MOLECULAR & GENERAL GENETICS, vol. 193, 1984 (Berlin, Heidelberg, New York)<br><br>M. HAHN et al. "Localized Muta-genesis in Rhizobium japonicum" pages 46-52<br><br>* Summary; page 50, left column * | 1,2,4, 5 | |
| D,X | MOLECULAR & GENERAL GENETICS, vol. 196, 1984 (Berlin, Heidelberg, New York)<br><br>M.N. JAGADISH et al. "Directed Transposon Tn5 mutagenesis and complementation in slow-growing, broad host range cowpea Rhizobium" pages 290-300<br><br>* Summary * | 1,2,4, 5 | |
| A,D | JOURNAL OF BACTERIOLOGY, vol. 127, no. 2, August 1976 (Washington DC)<br><br>R.J. MAIER et al. "Ineffective and Non-Nodulating Mutant Strains of Rhizobium japonicum" pages 763-769<br><br>* Page 763 * | 1-8 | |

TECHNICAL FIELDS SEARCHED (Int Cl 4)

The present search report has been drawn up for all claims

| Place of search | Date of completion of the search | Examiner |
|---|---|---|
| VIENNA | 29-07-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

| **European Patent Office** | **EUROPEAN SEARCH REPORT** | Application number |

EP 88106893.6

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A,D | JOURNAL OF BACTERIOLOGY, vol. 152, no. 1, October 1982 (Washington DC.) K.D. NOEL et al. "Rhizobium japonicum Mutants Defective in Symbiotic Nitrogen Fixation" pages 485-494 * Page 485; page 487, table 1 * ---- | 1-8 | |

TECHNICAL FIELDS SEARCHED (Int Cl 4)

The present search report has been drawn up for all claims

| Place of search | Date of completion of the search | Examiner |
|---|---|---|
| VIENNA | 29-07-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82